(19) **Europäisches Patentamt**

**European Patent Office**

**Office européen des brevets**

(11) **EP 1 153 907 B1**

(12) **EUROPEAN PATENT SPECIFICATION**

(45) Date of publication and mention
of the grant of the patent:
**06.10.2004 Bulletin 2004/41**

(51) Int Cl.7: **C07C 17/386**

(21) Application number: **01109907.4**

(22) Date of filing: **24.04.2001**

(54) **Purification process of pentafluoroethane (HFC-125)**

Verfahren zur Reinigung von Pentafluorethan (HFC-125)

Procédé de purification du pentafluoroéthane (HFC-125)

(84) Designated Contracting States:
**DE ES FR GB IT NL**

(30) Priority: **09.05.2000 IT MI001006**

(43) Date of publication of application:
**14.11.2001 Bulletin 2001/46**

(73) Proprietor: **Solvay Solexis S.p.A.**
**20121 Milano (IT)**

(72) Inventors:
• **Azzali, Daniele**
**20021 Bollate (MI) (IT)**
• **Basile, Giampiero**
**15100 Alessandria (IT)**

(74) Representative: **Jacques, Philippe et al**
**Solvay S.A.**
**Département de la Propriété Industrielle,**
**Rue de Ransbeek, 310**
**1120 Bruxelles (BE)**

(56) References cited:
**EP-A- 0 626 362**         **EP-A- 0 985 650**

**EP 1 153 907 B1**

**Description**

The present invention relates to a purification process of pentafluoroethane (HFC-125) containing as impurity chloropentafluoroethane (CFC-115).

Specifically the invention relates to a purification process of pentafluoroethane (HFC-125) starting from a HFC-125 and CFC-115 mixture by extractive distillation by using a third component which acts as an extracting agent.

HFC-125 is a hydrofluorocarbon used to replace, in mixture with other HFCs or HCFCs, some of the chlorofluorocarbons used in the past as refrigerants but not nowadays since dangerous to the stratospheric ozone. HFC-125, being indeed free from chlorine atoms, has no dangerous action on the atmospheric ozone, as required by the Montreal protocol and subsequent amendments.

It is known in the prior art that the industrial uses of HFC-125 require to obtain such compound with a high purity degree, in particular with low percentages of chloropentafluoroethane (CFC-115), preferably in an amount lower than 1000 ppm by moles, since the presence of chlorine causes incompatibility with the apparatus materials, for example corrosion.

Most industrial processes for the HFC-125 production lead to obtain CFC-115 as by-product. For example in the hydrofluorination process of perchloroethylene with HF in the presence of a fluorination catalyst, or the in catalytic reduction process of chloropentafluoroethane CFC-115 to pentafluoroethane HFC-125, a mixture of HFC-125 and CFC-115 is always obtained.

However, CFC-115 is a chlorofluorocarbon and as such it has a harmful action on the atmospheric ozone. On the other hand CFC-115 is not separable from HFC-125 by simple distillation, since these compounds form an azeotropic mixture. It is therefore necessary to subject HFC-125 contaminated by CFC-115, to particular treatments, able to eliminate CFC-115, for example by transforming it into useful products or easily separable from HFC-125. For this purpose, several purification processes of HFC-125 are described in the prior art.

Patent application WO 94/19301 describes a purification process of HFC-125 from CFC-115 wherein the azeotropic mixture 125/115 is subjected to different distillation steps wherein the pressure is changed in order to change the azeotrope composition. In the case of the azeotropic mixture 125/115, by lowering the pressure in the various distillation steps, compositions richer and richer in CFC-115 are obtained, pure HFC-125 is obtained at the column head. This purification process requires rather sophisticated technologies and therefore high costs.

WO 98/15511 and WO 97/03936 describe the addition to the azeotropic mixture 125/115 of a third component, such as for example difluoromethane (HFC-32) or 1,1,1-trifluoroethane (HFC-143a). Said fluids form a binary azeotrope with CFC-115, which has a different relative volatility with respect to the starting azeotrope so rendering possible the separation of HFC-125 from the azeotrope formed by azeotropic distillation. Also in this case the processes require rather sophisticated technologies and therefore high costs. Besides, the compounds used for the separation (HFC-32, HFC-143a) are compounds having a high value, therefore their recovery is necessary and additional steps are required.

Another purification process known in the prior art is the so called extractive distillation. When the two components of the mixture cannot be separated by conventional distillation, the mixture is added with a third component, the so called extracting agent. The chemical structure of the extracting agent is such to establish special interaction forces mainly with one of the two components to be separated.

The patent literature discloses the use of many chemical compounds as extracting fluids in the extractive distillation for mixture of HFC-125/CFC-115.

WO 96/06063 describes the use of an extracting solvent which is a polar organic compound containing oxygen and/or nitrogen having a dipole moment higher than at least 1 Debye. Alcohols, aldehydes, ketones, nitriles, acids, acid anhydrides, furans, ethers, esters can be mentioned.

WO 95/21148 describes an extractive distillation process wherein as extracting agent an alcohol, for example methanol or propanol is used. Tests carried out by the Applicant using methanol or diethylether as extracting agent have shown that a suitable separation efficiency is not obtained. In fact experimental values of relative volatility $\alpha$ higher than 0.5 are obtained (see later on). Therefore the use of an extractive column having a high number of plates, and therefore expensive, is necessary.

USP 5,087,329 describes the use of hydrofluorocarbons having 1-4 carbon atoms as extracting agents for the purification of HFC-125 from CFC-115. The drawback of this process is that expensive compounds are used as extracting agents whose preparation requires complex technologies.

WO 96/07627 teaches the use of extracting agents comprised in the class of perfluoroalkanes having 5 or more carbon atoms or the use of bromochloro-2,2,2-trifluoroethane. The same comments made for the previous patent are valid.

WO 95/21147 describes the use of hydrocarbons C5-C8, chlorocarbons and hydrochlorocarbons with at least 2 C atoms. The drawback of these extracting agents is that hydrocarbons are flammable and therefore they are to be used with caution in an industrial plant. Besides chlorocarbons and hydrochlorocarbons have an environmental impact.

WO 96/24569 describes the use of perchloroethylene as extractive distillation agent in the purification of HFC-

2

125 from CFC-115. The Applicant has found that, by using perchloroethylene as extracting solvent, a suitable separation efficiency is not reached, obtaining a relative volatility coefficient $\alpha$ comprised between 1 and 2 (see the comparative Examples).

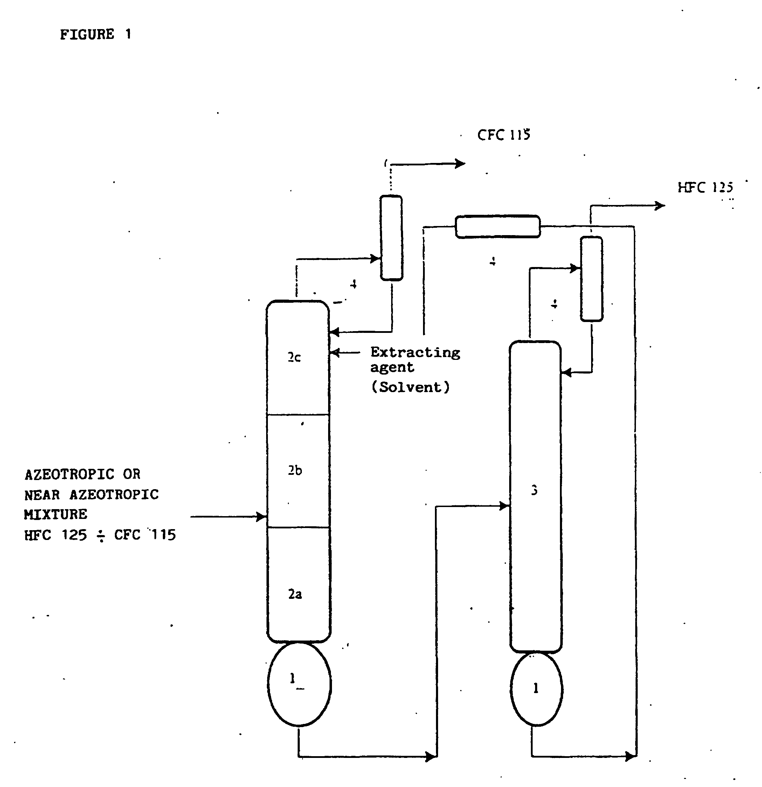

EP 626,362 discloses the use as extracting agents of paraffinic hydrocarbons, alcohols, ethers, esters and ketones. Acetone is particularly preferred as an extracting agent. It has been found by the Applicant that with acetone it is necessary to operate in the extractive column at a rather high column bottom temperature, about 52°C (see the comparative Examples), when the working pressure is of about 3 ata, typical operating pressure in an industrial plant. Consequently, in the column bottom there is a temperature approaching to the critical temperature of HFC-125 ($T_c$=75°C). This causes inconstant gaseous flows in the lowest exhaustion part (Figure 1, 2a) of the column, which notably worsen the possibility of its control. The presence of these inconstant gasous flows implies run operating problems of the extractive column with consequent worsening of the productivity. In fact, to obtain the desired purity of HFC-125, the production of the distilled HFC-125 must be recycled whenever it contains excessive amounts of CFC-115. This is a remarkable drawback from the industrial point of view since the productivity results greatly decreased.

EP-A-0985650 discloses the separation of HFC-125 from its mixtures with CFC-115 by extractive distillation with ethyleneglycol based compounds. The need was felt to use in the above described separation process an extracting solvent such to guarantee a high separation efficiency, eliminating the drawbacks of the extracting agents used in the prior art and making in the same time more controllable from the operating point of view the extractive distillation column, i.e. without frequent recycle of the distilled HFC-125 for obtaining the product with the desired purity.

An object of the present invention in an extractive distillation process for separating pentafluoroethane (HFC-125) from a mixture of pentafluoroethane (HFC-125) and chloropentafluoroethane (CFC-115) wherein as extracting agent an acetal having the formula

$$R_1\text{-O-CH}_2\text{-O-R}_2 \qquad\qquad (I)$$

is used, wherein $R_1$, $R_2$, equal to or different from each other, are a linear or branched when possible C1-C3 alkyl.

Also mixtures of the above acetals can be used. Dimethoxymethane ($R_1$=$R_2$=$CH_3$) is particularly preferred.

It has been surprisingly found that by using the compounds of formula (I) as extracting solvents, it is possible to obtain a high separation efficiency, without the above mentioned problems of inconstant gaseous flows in the extractive column.

The obtainment of said results is surprising and unexpected if one considers that with the ethers, for example by using diethylether $C_2H_5$-O-$C_2H_5$ as extracting agent (see the comparative Examples), a suitable efficiency separation is not obtained, obtaining a relative volatility coefficient $\alpha$ higher than 0.5. Without to be bound to any theory, this could be explained by the fact that the extracting agent of the invention does not belong to the ether class, but to the acetal one. As known to the skilled man in the field, the typical reactions of the acetals differ from those of the ethers. In fact, for example, acetals are not subject to acid or alkaline hydrolysis and they do not form peroxides as it happens for ethers which are just identifiable by this kind of reactions.

Besides the boiling point at atmospheric pressure of the preferred compound, dimethoxymethane, is of 42.3°C, therefore very close to room temperature. Therefore it is very well suitable to the use as extracting solvent in the process of the present invention, since it is possible to use in the extractive column a working pressure of the order of the atmospheric one.

It is defined hereinafter what it is meant by volatility ratio or relative volatility. As known, in azeotropic or near azeotropic binary compositions the two components show a volatility ratio very close to the unit. Such ratio is defined from the following relation:

$$\alpha = (y_a/x_a)/(y_b/x_b) \qquad\qquad (1)$$

wherein

$y_a$=  molar fraction of the more volatile component in the vapour
$X_a$=  molar fraction of the more volatile component in the liquid
$y_b$=  molar fraction of the lower volatile component in the va pour
$x_b$=  molar fraction of the lower volatile component in the liquid.

When a third component is added, which causes strong interactions mainly with one of the two components to be separated, it is noticed that such ratio moves from the unit with values lower than or higher than 1, making it possible the separation by distillation of one of the two components while the other remains in mixture with the extracting agent. Such separation is said extractive distillation and it is much more effective as the relative volatility in the presence of the extracting compound more differs from 1. It has been found by the Applicant that the process of the invention allows to obtain with the compounds of formula (I) volatility ratios lower than 0.5 at the working temperatures of the extractive distillation column bottom. In any case the component extracted by the extracting agent and mixed thereto is separated without difficulty by a second distillation. This second distillation has the efficiency which normally regulates this kind of separation and depends on the boiling temperatures and generally on the physical properties of the extracting compound compared with those of the extracted component. In the extractive distillation of the invention, the component not extracted from the solvent comes out from the extractive column head.

The purification process of the invention starting from an azeotropic or near azeotropic mixture of HFC-125 and CFC-115 is schematized in Figure 1, wherein the extractive distillation column and the subsequent distillation column are shown, from which HFC-125 is obtained at the column head and the extracting agent from the column bottom. In Figure 1, reference 1 indicates the boilers, reference 2 the extractive distillation column, references 2a, 2b, 2c show respectively the exhaustion, absorption and recovery sections, reference 3 the second distillation column and reference 4 the heat exchangers.

The higher the amount of extracting agent with respect to the fed amount of azeotropic or near azeotropic binary mixture, the better the efficiency of the extractive distillastillation. It has been noticed that to obtain a favourable volatility ratio (lower than 0.5) a ratio by weight between the extracting agent and the fed mixture 125/115 in the range 1-10 can be used. In particular, to obtain a better separation efficiency, it is advantageous to use a ratio between the extracting agent and the fed mixture in the range 1.3-3.5.

Generally the higher the average temperature at which the extractive distillation carried out, the worse the efficiency of the extractive distillation. For example, by using dimethoxymethane as extracting agent, the working temperature of the column bottom is in the range 25°-45°C, at a working pressure comprised between 101,3 and 1013 kPa (1 and 10 ata). Said working temperature of the column bottom is clearly lower than the critical temperature of HFC-125 (Tc=75°C), wherefore there are no inconstant gaseous flows in the exhaustion part (Figure 1, 2a) of the extractive distillation column, which notably worsen the possibility of its control.

Besides, contrary to what reported in the patent literature, see to this purpose patent application WO 96/06063, the Applicant has surprisingly found a class of extracting agents which even though it has a low polarity (0.74 Debye at T=25°C for the dimethoxymethane), shows a high separation efficiency.

The present invention allows to use as extracting agents easily available and cheap solvents since widely diffused in the technical and cosmetic formulations for aerosol as solvents of the propellant. Another advantage is given by the use of non corrosive, non dangerous to the environment fluids, having zero Ozone Depleting Potential (ODP) with low Volatile Organic Concentration (VOC). Besides, the solvents of the invention are soluble with the azeotropic or near azeotropic mixture to be separated to such an extent that in no case, by varying the HFC 125/CFC 115 ratio, any miscibility lack in a wide range of temperatures and anyhow in the whole range of useful temperatures has been observed.

The extracting agents of the present invention show a high extractive efficiency, such that the above defined volatility ratio becomes lower than 0.5 under the above defined operating conditions. For example, the relative volatility α value in the presence of dimethoxymethane results comprised between 0.34-0.49 (see Example 3) in the temperature range 25°-40°C. Column bottom temperatures in this temperature range can therefore be used to obtain a high extraction efficiency.

By the invention process it is possible to obtain a good separation of CFC-115 from HFC-125, as it results from the Examples, obtaining HFC-125 having a high purity with a final content of CFC-115 lower than 0.1% by moles (1000 ppm).

The present invention will be better illustrated by the following Examples, which have a merely indicative but not limitative purpose of the scope of the invention itself.

**EXAMPLES**

EXAMPLE 1

Some essential measurements are carried out on the binary mixture HFC-125/CFC-115. The mixture HFC-125/CFC-115 is introduced into a glass cell, the volume of which is 15 cm$^3$, suitably constructed for the measurements of the liquid-vapour and liquid-liquid equilibria calculating the respective amounts from the cell weight difference. The purpose of the test is to measure the boiling temperature of the saturated liquid in connection with the reciprocal amounts of HFC-125/ CFC-115. These measurements can be advantageously carried out at increasing pressures for each prefixed composition, by simply observing the temperature at which the prefixed pressure is reached after a suitable period of equilibrium. The mixture composition in liquid phase is that obtained by weight differences as above said and then applying the correction necessary to take into account the amount of fluid passed into vapour phase. The glass cell is dipped and thermostated in a steel bath containing 80 litres of thermostatic fluid. The bath has a side equipped with a glass window to see inside the cell if the liquid is homogeneous or if it shows separation of two liquid phases. The temperature of the sample inside the cell is measured by a thermoresistance placed in the bath in the immediate vicinity to the cell itself. The bath is continuously stirred in order not to show temperature gradients. The cell has a system for the magnetic stirring of the sample contained therein. It has an outlet for the pressure measurement which is transmitted to an external membrane the equilibrium of which is restored by an equilibrating system of nitrogen pressure.

The measurement of the boiling temperature allows to identify azeotropic or near azeotropic compositions, since such compositions show a minimum with respect to the boiling temperatures of the pure components. The results are reported in Table 1.

It is clear that the minimum of temperature (-49.22°C) measured at 1 bar pressure corresponds to an azeotropic composition HFC-125/CFC-115 equal to 80/20 by mole. The minimum of temperature (13.06°C) measured at 10 bar pressure corresponds instead to an azeotropic composition HFC-125/CFC-115 equal to 90/10 by mole. The Example points out the existence of real azeotropy, which therefore justifies the use of an extractive distillation for separating the components.

TABLE 1

| Liquid molar fraction HFC-125 | Boiling temperature (°C) (P=1 bar) | Boiling temperature (°C) (P= 10 bar) |
|---|---|---|
| 0.0 | -39.19 | 28.93 |
| 0.1 | -42.89 | 24.49 |
| 0.2 | -45.19 | 21.20 |
| 0.3 | -46.70 | 18.73 |
| 0.4 | -47.72 | 16.85 |
| 0.5 | -48.41 | 15.43 |
| 0.6 | -48.88 | 14.37 |
| 0.7 | -49.15 | 13.63 |
| 0.8 | -49.22 | 13.18 |
| 0.9 | -49.01 | 13.06 |
| 1.0 | -48.41 | 13.31 |

EXAMPLE 2

New measurements on the binary mixture HFC-125/CFC-115 are carried out to evaluate the relative volatility in the saturated liquid zone and in the temperature range meaningful for the extractive distillation of the present invention. For the calculation it is necessary to experimentally measure the compositions of the liquid and vapour phases in equilibrium. The cell is equipped for the sampling and the analysis in line of the vapour phase by gaschromatography, while for the liquid phase the composition is calculated as described in Example 1. The volatility ratio $\alpha$ is obtained from the following equation:

$$\alpha = (y_a/x_a)/(y_b/x_b) \tag{1}$$

wherein:

$y_a$= molar fraction of the more volatile component in the vapour
$x_a$= molar fraction of the more volatile component in the liquid
$y_b$= molar fraction of the lower volatile component in the vapour
$x_b$= molar fraction of the lower volatile component in the liquid.

Such ratios $\alpha$ measured in the whole range of CFC-115 molar fractions comprised bewtween 0 and 1 are described in Table 2. It is noticed that close to an azeotrope ($\alpha$=1), there is also a wide range of near azeotropic compositions having a volatility ratio very close to 1. It is confirmed that the compositions show azeotropic behaviour starting from molar ratios of HFC-125 equal to 0.7 and that the separation anyway appears difficult, for example, in the composition range having molar fractions of HFC-125 higher than 0.5.

The Example identifies therefore the composition range for which it is suitable to operate by extractive distillation.

TABLE 2

| Liquid molar fraction $X_{125}$ | Relative volatility $\alpha$ | | | | |
|---|---|---|---|---|---|
| | T=15°C | T=25°C | T=30°C | T=40°C | T=60°C |
| 0.1 | 1.95 | 1.84 | 1.78 | 1.68 | 1.41 |
| 0.2 | 1.78 | 1.70 | 1.64 | 1.56 | 1.32 |
| 0.3 | 1.63 | 1.57 | 1.52 | 1.46 | 1.25 |
| 0.4 | 1.50 | 1.45 | 1.42 | 1.36 | 1.18 |
| 0.5 | 1.38 | 1.34 | 1.32 | 1.28 | 1.13 |
| 0.6 | 1.27 | 1.25 | 1.23 | 1.21 | 1.09 |
| 0.7 | 1.17 | 1.16 | 1.15 | 1.14 | 1.06 |
| 0.8 | 1.08 | 1.08 | 1.08 | 1.07 | 1.03 |
| 0.9 | 0.99 | 1.00 | 1.00 | 1.01 | 1.01 |

EXAMPLE 3

The same measurements of the volatility ratio $\alpha$ indicated in Example 2 are carried out, when to the azeotropic or near azeotropic binary mixture HFC-125/CFC-115 dimethoxymethane is added as extracting agent.

The relative volatility values measured in the presence and in absence of the extracting agent of the invention are reported in Table 3. The comparison among the values of $\alpha$, made at different temperatures and compositions of the mixture 125/115, clearly points out the extracting (or solvent) effect of dimethoxymethane. This is shown from the values in Table 3, wherein:

$\alpha$ = relative volatility in absence of dimethoxymethane
$\alpha'$ = relative volatility in the presence of dimethoxymethane

TABLE 3

| Mixture composition | | Temp. (°C) | Extracting agent (% by wt.) | Extracting agent presence $\alpha'$ | Extracting agent absence $\alpha$ |
|---|---|---|---|---|---|
| $X_{125}$ | $X_{115}$ | | | | |
| 0.590 | 0.410 | 25 | 57.0 | 0.45 | 1.25 |
| 0.985 | 0.015 | 25 | 57.0 | 0.34 | 1.00 |
| 0.590 | 0.410 | 40 | 57.0 | 0.49 | 1.21 |
| 0.985 | 0.015 | 40 | 57.0 | 0.38 | 1.01 |
| 0.590 | 0.410 | 65 | 33.0 | 0.72 | 1.07 |
| 0.985 | 0.015 | 65 | 33.0 | 0.61 | 1.01 |

EXAMPLE 4 (comparative)

The same measurements of the volatility ratio $\alpha$ indicated in Example 3 are carried out, when as extracting agent methanol, diethylether, perchloroethylene are used. The relative volatility values measured in the presence of said extracting agents are reported in Table 4.

The comparison among the values of $\alpha$, made at different temperatures and compositions of the mixture 125/115, points out that by using as extrating agent methanol or diethylether, the relative volatility is in any case higher than 0.5, while by using as extracting agent perchloroethylene, the relative volatility is lower than 2.

TABLE 4

| EXTRACTING AGENT = METHANOL | | | | |
|---|---|---|---|---|
| Feeding | | Temp. (°C) | Extracting agent (% by wt.) | α |
| $X_{125}$ | $X_{115}$ | | | |
| 0.985 | 0.015 | 25 | 57.0 | 0.68 |
| 0.985 | 0.015 | 65 | 33.0 | 0.72 |
| EXTRACTING AGENT = DIETHYLETHER | | | | |
| 0.590 | 0.410 | 25 | 57.0 | 0.62 |
| EXTRACTING AGENT = PERCHLOROETHYLENE | | | | |
| 0.560 | 0.440 | 15 | 79.0 | 1.81 |
| 0.880 | 0.120 | 31 | 92.7 | 1.61 |
| 0.590 | 0.410 | 85 | 96.8 | 1.55 |
| 0.880 | 0.120 | 33 | 93.2 | 1.32 |

EXAMPLE 5

With reference to Figure 1, a flow rate equal to 3.5 kg/h of raw HFC-125 containing CFC-115 at a concentration 2.35% by mole, is fed into a stainless steel extractive column (2) having a diameter of 50 mm.

The column has a theoretical plate number equal to 36 plates and feeding is placed in correspondence with the 15th plate starting from the top. A dimethoxymethane flow having a flow-rate of 12.3 Kg/h, is fed in correspondence of the 5th plate starting from the top. The column under the working conditions operates at a pressure of 303,9 kPa (3 ata) and the reflux ratio at the condenser is equal to 34.

It is noticed that, under steady conditions, the temperature at the column head is equal to -22°C, while at the bottom of the extractive column a temperature equal to about 43°C is measured. No inconstant gaseous flows in the exhaustion part are evident (Figure 1, 2a).

The residue taken from the column bottom, at a flow-rate equal to 15.6 Kg/h, contains purified HFC-125 and the extracting agent dimethoxymethane. Said residue, after having been partially evaporated in the boiler (1), is fed into the distillation column (3), from which purified HFC-125 is recovered from the head and from the column bottom the extracting agent which can be used again in the extractive column (2). HFC-125 having a high purity degree is thus obtained, containing only 0.075% by mole of CFC-115 (750 ppm by mole). The compositions in % by mole of the feeding, of the distillate and of the bottom residue of the extractive column are indicated in Table 5.

This Example shows that the process of the invention allows to obtain HFC-125 having a high purity degree and meanwhile it points out that surprisingly inconstant gaseous flows in the exhaustion part do not form, wherefore the operating control problems of the extractive column are minimized.

TABLE 5

| | Feeding | Distillate | Residue |
|---|---|---|---|
| HFC-125 (% by mole) | 97.65 | 57.45 | 14.492 |
| CFC-115 (% by mole) | 2.35 | 42.55 | 0.011 |
| dimethoxymethane (% by mole) | - | negligible | 85.497 |

EXAMPLE 6 (comparative)

Raw HFC-125 containing CFC-115 at a concentration 2.35% by mole, is fed with the same flow-rate of Example 5 to the same distillation column. Feeding is placed at the same height (15th plate). The column is made to work at the same working pressure (303,9 kPa) (3 ata) and using the same reflux ratio (34), with the difference that no extracting solvent is fed.

No separation effect of the components constituting the fed mixture is practically obtained, as it is shown from the values reported in Table 6, wherein the compositions % by mole of the feeding, of the distillate and of the bottom residue of the extractive column are reported.

TABLE 6

|  | Feeding | Distillate | Residue |
|---|---|---|---|
| HFC-125 (% by mole) | 97.65 | 96.83 | 97.88 |
| CFC-115 (% by mole) | 2.35 | 3.17 | 2.12 |

EXAMPLE 7 (comparative)

The extractive distillation described in Example 5, is carried out by using acetone as extracting agent instead of dimethoxymethane.

Under the optimal conditions of feeding and of the extracting agent flow-rate, the same raw HFC-125 of Example 5 (2.35% by mole of CFC-115) is fed into the extractive column. A separation of CFC-115 from HFC-125 equal to 0.076% by mole of CFC-115 (760 ppm by mole) is obtained, therefore comparable with that obtained by using dimethoxymethane. However, the use of acetone determines, along the whole extractive column, a more marked increase of the temperature, wherefore on the column bottom a temperature of about $52°C$ is determined.

As a consequence in the column bottom zone the operating temperature gets near the critical temperature of HFC-125 (equal to $75°C$), and this often causes inconstant gaseous flows in the exhaustion part (Figure 1, 2a) during the normal offset periods of the temperature regulation system. As it is known to the skilled man in the art, the presence of these inconstant gaseous flows implies operating control problems of the extractive column with consequent worsening of the productivity.

**Claims**

1. An extractive distillation process for separating pentafluoroethane (HFC-125) from a mixture containing pentafluoroethane (HFC-125) and chloropentafluoroethane (CFC-115), wherein as extracting agent an acetal having the formula

$$R_1\text{-O-CH}_2\text{-O-R}_2 \qquad \text{(I)}$$

is used, wherein $R_1$, $R_2$, equal to or different from each other, are a linear or branched when possible C1-C3 alkyl.

2. An extractive distillation process according to claim 1, wherein as extracting agent dimethoxymethane is used.

3. An extractive distillation process according to claims 1-2, wherein the ratio by weight between the extracting agent and the fed mixture 125/115 is in the range 1-10.

4. An extractive distillation process according to claim 3, wherein the ratio by weight between the extracting agent and the fed mixture 125/115 is in the range 1.3-3.5.

5. An extractive distillation process according to claims 1-4, wherein the working temperature of the column bottom is in the range $25°C$-$45°C$ and the working pressure is comprised between 101,3 and 1013 kPa (1 and 10 ata).

**Patentansprüche**

1. Extraktives Destillationsverfahren zur Abtrennung von Pentafluorethan (HFC-125) aus einem Pentafluorethan (HFC-125) und Chlorpentafluorethan (CFC-115) enthaltenden Gemisch, worin als Extraktionsmittel ein Acetal mit der Formel

$$R_1\text{-O-CH}_2\text{-O-R}_2 \qquad \text{(I)}$$

verwendet wird, worin $R_1$ und $R_2$, die gleich oder voneinander verschieden sind, ein lineares oder, wo dies möglich ist, ein verzweigtes $C_1$-$C_3$-Alkyl darstellen.

**2.** Extraktives Destillationsverfahren nach Anspruch 1, worin als Extraktionsmittel Dimethoxymethan verwendet wird.

**3.** Extraktives Destillationsverfahren nach den Ansprüchen 1 bis 2, worin das Gewichtsverhältnis zwischen dem Extraktionsmittel und dem zugeführten 125/115-Gemisch im Bereich 1 bis 10 liegt.

**4.** Extraktives Destillationsverfahren nach Anspruch 3, worin das Gewichtsverhältnis zwischen dem Extraktionsmittel und dem zugeführten 125/115-Gemisch im Bereich 1,3 bis 3,5 liegt.

**5.** Extraktives Destillationsverfahren nach den Ansprüchen 1 bis 4, worin die Arbeitstemperatur des Kolonnensumpfes im Bereich 25°C bis 45°C liegt und der Arbeitsdruck zwischen 101,3 und 1.013 kPa (1 und 10 ata) liegt.

**Revendications**

**1.** Procédé de distillation extractive pour séparer le pentafluoroéthane (HFC-125) d'avec un mélange contenant du pentafluoroéthane (HFC-125) et chloropentafluoroéthane (CFC-115) dans lequel on emploie un acétal ayant pour formule

$$R_1\text{-O- }CH_2\text{-O-}R_2 \qquad\qquad\qquad \text{(I)}$$

comme agent d'extraction, dans laquelle $R_1$, $R_2$, identiques ou différents l'un de l'autre, représentent un groupe alkyle en $C_1$ à $C_3$, linéaire ou ramifié si possible.

**2.** Procédé de distillation extractive selon la revendication 1, dans lequel on emploie le diméthoxyméthane comme agent d'extraction.

**3.** Procédé de distillation extractive selon les revendications 1 à 2, dans lequel le rapport pondéral entre l'agent d'extraction et le mélange 125/115 introduit est dans la plage de 1 à 10.

**4.** Procédé de distillation extractive selon la revendication 3, dans lequel le rapport pondéral entre l'agent d'extraction et le mélange 125/115 introduit est dans la plage de 1,3 à 3,5.

**5.** Procédé de distillation extractive selon les revendications 1 à 4, dans lequel la température de travail du fond de la colonne est dans la plage de 25°C à 45°C et la pression de travail est comprise entre 101,3 et 1013 kPa (1 et 10 atm).

FIGURE 1

FIGURE 1